# EUROPEAN PATENT APPLICATION

(11) **EP 4 682 260 A1**
(43) Date of publication of application: **21.01.2026**
(21) Application number: 24189745.3
(22) Date of filing: 19.07.2024
(51) Int. Cl.: C12P 7/22, A61K 8/34, A61K 8/35, C12P 7/26, C12R 1/225

(54) **ANTIMICROBIAL LACTOBACILLUS FERMENT**

(71) Applicant: Symrise AG, 37603 Holzminden Niedersachsen (DE)
(72) Inventor: Herrmann, Martina, 31789 Hameln (DE); Joppe, Holger, 37586 Dassel (DE); Koch, Christin, 32760 Detmold (DE); Schmaus, Gerhard, 37671 Höxter-Bosseborn (DE); Pesaro, Manuel, 1214 Vernier (CH); Dormeyer, Miriam, 37603 Holzminden (DE)

(57) **Abstract**

The invention is directed to a natural method for producing an antimicrobial composition comprising piceol which is produced from picein by fermentation of picein-containing plant spruce needles extracts using Lactobacillus rhamnosus. Moreover, the invention relates to an antimicrobial composition comprising at least one microorganism and piceol. In addition, the invention concerns an antimicrobial composition comprising piceol and picein, wherein piceol is in an excess of picein.

## Description

The present invention generally relates to the subject of natural antimicrobial agents. In particular, the invention is directed to a natural method for producing an antimicrobial composition, preferably comprising piceol. Moreover, the invention relates to an antimicrobial composition comprising at least one microorganism and piceol. In addition, the invention concerns an antimicrobial composition comprising piceol and picein, wherein piceol is in an excess of picein. The invention further relates to the use of a microorganism, preferably a lactobacillus species, for producing an antimicrobial composition from picein. Additional aspects of the invention are presented in the attached claims as well as in the description below.

Microbial contaminants can not only cause infections, skin irritations and allergic reactions, they also significantly decrease the shelf-life of personal care products. If such products are not sufficiently preserved, microbial contamination manifests in e.g. smell, discoloration, and visible mould growth. Thus, antimicrobial agents are added to personal care products. By means of various chemical and biological processes, microorganisms can be stopped from deteriorating product quality, thereby enhancing the shelf-life.

For decades antimicrobial agents for personal care products have largely been derived from crude oil sources since they are cheap and easy to produce.

EP0167286A1, for instance, teaches a process for producing crude 4-HAP. It describes the production of hydroxy aromatic ketones such as 4-hydroxyacetophenone (4-HAP) by the Friedel-Crafts acylation of monocyclic phenolic reactants.

DE2653601 A1 discloses the hydrogen fluoride-catalyzed acylation of phenolic compounds such as phenol itself with an acyl halide such as acetyl chloride to form hydroxy aromatic ketones.

EP0069597 B1 teaches the preparation of p-phenoybenzoyl compounds by reacting diphenyl ether and an appropriate acyl compound such as acetic anhydride in the presence of hydrogen fluoride.

The educts of these processes are mainly of petrochemical origin. The produced antimicrobials are thus crude-oil derived.

Increasingly, however, consumers demand that products comprise exclusively "natural" antimicrobial agents. The choice of personal care products shall contribute to reaching a "circular economy". As a result, natural raw materials for antimicrobial agents steadily gain momentum.

Despite being widely and commonly used, the terms "natural" and "sustainable" have not been defined officially in cosmetics until enacting ISO 16128 in 2016 and 2017. Having two separate parts, the first part (ISO 16128-1) defines the terms "natural" and "derived natural" ingredients. The second part (ISO 16128-2) offers a guideline to calculate the "naturalness" of cosmetic ingredients. To comply with ISO 16128-2 a value of at least 0.9 from a maximum of 1.0 shall be reached. This can be achieved if the ingredient is derived from a "sustainable" source. "Sustainable" in this context means a material whose usage does not lead to the depletion of a limited resource (e.g. petroleum or fossil fuel). Using such materials means the usage of a carbon source, which has existed only since recently and is renewable.

To distinguish between a petroleum based - and a product which is from a renewable source, the skilled person knows various techniques including the evaluation of carbon-12/carbon-13 and/or hydrogen-1/hydrogen-2 ratios and radiocarbon dating. Being an unstable isotope of carbon known as ¹⁴C, radiocarbon emits beta particles until finally decaying to the more stable ¹⁴N. Since crude oil derived products originate from carbon sources dating back millions of years, radiocarbon has decayed. Considering that the amount of radiocarbon in "sustainable" current plant sources is known, it can hence be calculated how much of the total organic carbon is petrochemically derived.

Besides being sustainable, antimicrobial agents must obviously fulfil a multitude of further requirements, e.g., remain effective for an extended period, be compatible with the final formulation of a personal care product and have broad spectrum antimicrobial activity. Thus, modern personal care products must fulfil a diverse profile of requirements driven by environmental and efficacy concerns. The availability of products achieving such standards is highly limited. In the state of the art, there is thus an ongoing and steadily increasing need for effective, multifunctional antimicrobial agents derived from sustainable, natural sources.

One antimicrobial agent widely used in the cosmetic industry is 4-hydroxyacetophenone (4-HAP), which is also called "piceol". Derivable from its inactive precursor "picein," the compound occurs in several plant genera such as the needles of Norway sprues (Picea abies). Piceol has many applications, for example the stabilisation of pharmaceutical and cosmetic compositions.

4-HAP is described by the CAS number 99-93-4 (as listed by the European Chemicals Agency) and corresponds to the following structure:

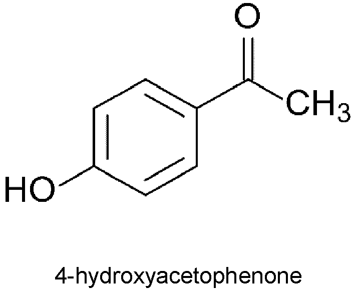

Typically, when 4-hydroxyacetophenone is produced on an industrial scale, the compound is obtained by a chemical synthesis. Briefly, phenol reacts with acetic acid or acetic anhydride in the presence of water-free hydrogen fluoride (HF) or hydrofluoric acid to form phenyl acetate, which is transformed to 4-hydroxyacetophenone by Fries rearrangement. However, hydrogen fluoride and hydrofluoric acid are toxic substances. Thus, chemical production processes require complex safety mechanisms, special equipment, and specific conditions for waste disposal. Hence, natural, biotechnological production methods are of increasing interest. Unfortunately, the production of antimicrobial compositions from natural sources is challenging due to a low yield.

A first, primary object of the present invention was thus to provide an environmentally friendly method for producing an antimicrobial composition from exclusively renewable sources, preferably with enhanced antimicrobial activity. A further object of the invention was to provide a natural, effective antimicrobial composition. Moreover, it was an object of the invention to improve the antimicrobial properties of natural plant extracts. Additional objects of the present invention can be derived from the attached claims and the description below.

The primary object of the invention is achieved by the method according to claim 1. Preferable embodiments are subject of the dependent claims, the description below, and the figures. In a first aspect, the invention relates to a method for producing an antimicrobial composition, preferably comprising piceol, comprising the following steps:
- providing a growth medium comprising picein and at least one microorganism
- fermenting the mixture comprising picein and said microorganism, thereby forming the antimicrobial composition, preferably comprising piceol
- optionally removing the cells of the microorganism from the mixture
- and/or optionally inactivating the microorganism

Surprisingly, it was found that the method according to claim 1 forms a natural antimicrobial composition with a significantly enhanced yield of piceol. In turn, the concentration of its less effective precursor, picein, is significantly decreased compared to its concentration in the natural, untreated plant extract. The invention thus constitutes an environmentally friendly alternative to producing antimicrobial agents by conventional, crude oil-based means. In a preferred embodiment, the method provides a piceol content of at least 200 ppm, more preferably of at least 500 ppm, even more preferably of at least 1000 ppm, and most preferably of at least 3000 ppm piceol. In a further preferred embodiment, the invention provides an antimicrobial composition with a ratio of piceol to picein of at least 2:1, more preferred of at least 4:1, even more preferred of at least 10 : 1, further preferred 40:1 and most preferred of at least 100 : 1. In a further preferred embodiment, the percentage of piceol with respect to the sum of piceol and picein amounts to at least 95 wt% and even more preferably to at least 97 wt%. Most preferably, 100 wt% piceol is produced.

In a preferred embodiment, the picein is a picein extract, extracted from a plant selected from the group consisting of Spruce, Salix, Phagnalon Rupestre, Rhodiola Rosea, Poacynum hendersonii, Baccharis magellanica or Vauquelinia. Such plant extracts contain picein and piceol. Picein, the precursor of piceol showing less antimicrobial efficacy, is present in the untreated extracts to a higher degree than piceol. In a preferred aspect, the picein extract is extracted from a Spruce needle and/or a Baccharis magellanica plant.

Lactic acid bacteria (LAB) have found widespread usage as probiotics and various health-related applications, i.e. the irritable bowel syndrome (IBS). Preferably, the microorganism in the method according to the invention is a Lactobacillus species, preferably selected from the group consisting of Lactobacillus acetotolerans, Lactobacillus acidophilus, Lactobacillus agrestimuris, Lactobacillus alvei, Lactobacillus amylolyticus, Lactobacillus amylovorus, Lactobacillus animate, Lactobacillus apis, Lactobacillus bombicola, Lactobacillus brevis, Lactobacillus brevisimilis, Lactobacillus casei, Lactobacillus catenefornis, Lactobacillus colini, Lactobacillus corticis, Lactobacillus crispatus, Lactobacillus delbrueckii, Lactobacillus equicursoris, Lactobacillus faeni, Lactobacillus fermentum, Lactobacillus fornicalis, Lactobacillus gallinarum, Lactobacillus gasseri, Lactobacillus gigeriorum, Lactobacillus guizhouensis, Lactobacillus halophilus, Lactobacillus hamsteri, Lactobacillus helsingborgensis, Lactobacillus helveticus, Lactobacillus hominis, Lactobacillus huangpiensis, Lactobacillus iatae, Lactobacillus iners, Lactobacillus insectis, Lactobacillus intermedius, Lactobacillus intestinalis, Lactobacillus isalae, Lactobacillus japonicus, Lactobacillus jensenii, Lactobacillus johnsonii, Lactobacillus juensis, Lactobacillus kalixensis, Lactobacillus kefiranofaciens, Lactobacillus kimbladii, Lactobacillus kitasatonis, Lactobacillus kullabergensis, Lactobacillus laiwuensis, Lactobacillus larvae, Lactobacillus leichmannii, Lactobacillus letivazi, Lactobacillus melliventris, Lactobacillus mobilis, Lactobacillus mulieris, Lactobacillus nasalidis, Lactobacillus panisapium, Lactobacillus paracasei, Lactobacillus paragasseri, Lactobacillus paraplantarum, Lactobacillus pasteurii, Lactobacillus plantarum, Lactobacillus porci, Lactobacillus psittaci, Lactobacillus rennanquilfy, Lactobacillus reuteri, Lactobacillus rizhaonensis, Lactobacillus rhamnosus, Lactobacillus rodentium, Lactobacillus rogosae, Lactobacillus sakei, Lactobacillus salivarius, Lactobacillus selangorensis, Lactobacillus sucicola, Lactobacillus taiwanensis, Lactobacillus terrae, Lactobacillus ultunensis, Lactobacillus vermiforme, Lactobacillus xujianguonis and Lactobacillus xylocopicola.

In a further preferred aspect, the microorganism is selected from the group consisting of Lactobacillus plantarum, Lactobacillus rhamnosus, Lactobacillus brevis, Lactobacillus paraplantarum, Lactobacillus reuteri, Lactobacillus pentosus, Lactobacillus buchneri, Lactobacillus sucicola, Lactobacillus bombi, Lactobacillus gasseri and Lactobacillus rodentium. In yet a further preferred aspect, the microorganism is a Lactobacillus rhamnosus strain. In a most preferred embodiment, the natural extract is a spruce needle extract and/or an extract of the plant Baccharis magellanica and the microorganism according to the invention is a Lactobacillus rhamnosus strain.

In a further preferred embodiment, the method comprises the step of downstream processing of the formed antimicrobial composition, preferably wherein the downstream processing comprises a method selected from the group consisting of centrifugation, crystallization, solid-phase adsorption, filtration, reverse osmosis, precipitation, decantation, liquid/liquid extraction, vacuum distillation, falling film distillation, melt crystallization, drying and/or combinations thereof.

In a preferred embodiment, the microorganisms are filtered out. In a further preferred embodiment, heat-inactivation, e.g. by tyndallization or pasteurization, or cell lysis is used as a means for inactivating said microorganisms. Further preferably, the antimicrobial composition is recovered from said mixture.

In a further preferred aspect, the formed antimicrobial composition is substantially non-petrochemical. Said composition is particularly advantageous due to its environmental friendliness and its naturalness, thereby meeting increasing consumer needs and complying with ISO 16128. The composition is considered "substantially non-petrochemical", if at least 75 %, preferably at least 80 %, further preferably at least 85 %, particularly preferably at least 90 %, even further preferably at least 95 % of its carbon is non-petrochemically-derived carbon.

Most preferably, all carbon of piceol in the composition is non-petrochemically-derived carbon based on the total carbon content of the piceol in the composition. Preferably, for the assessment radiocarbon dating is used.

In a further preferred aspect, the method comprises that the plant extracts are extracted by water extraction leading to a transparent antimicrobial composition.

In another aspect, the invention relates to an antimicrobial composition, preferably comprising piceol, obtained or obtainable by a method comprising or consisting of the steps as defined in any of the method claims.

It was found that using the method according to the invention provides piceol from non-petrochemically sources. Hence, the produced piceol is also non-petrochemically-derived. The natural antimicrobial composition obtained by the method comprises a significantly enhanced yield of piceol.

In still another aspect, the invention relates to an antimicrobial composition comprising or consisting of at least one microorganism, preferably a Lactobacillus species and piceol, preferably in an excess amount compared to picein. Optionally, said composition comprises picein and, also optionally, further plant-derived substances.

In a preferred aspect, said antimicrobial composition comprises a piceol content of at least 200 ppm, more preferably of at least 500 ppm, even more preferably of at least 1000 ppm, and most preferably of at least 3000 ppm. In a further preferred embodiment, the invention provides an antimicrobial composition with a ratio of piceol to picein of at least 2:1, more preferred of at least 4:1, even more preferred of at least 10 : 1, further preferred 40:1 and most preferred of at least 100 : 1. In yet another preferred embodiment, the percentage of piceol with respect to the sum of piceol and picein amounts to at least 95 wt% and even more preferably to at least 97 wt%. Most preferably, 100 wt% piceol is produced.

In a preferred aspect, the composition comprises at least 75 wt%, preferably at least 80 wt%, further preferably at least 85 wt%, particularly preferably at least 90 wt%, even further preferably at least 95 wt% non-petrochemically-derived carbon based on the total carbon content of the piceol in the composition. Most preferably, all carbon of the piceol in the composition is non-petrochemically-derived carbon based on the total carbon content of the piceol in the composition.

In a further preferred aspect, the antimicrobial composition contains further plant-derived substances, preferably selected from the group consisting of organic acids such as shikimic acid, quinic acid, citric acid, malic acid, succinic acid, threonic acid and lactic acid. Said plant-derived substances have antibacterial activity leading to a further improved antimicrobial efficacy of the antimicrobial composition. Additionally, said substances exhibit exfoliating and moisturizing effects on skin.

In a preferred aspect, the antimicrobial composition comprises a Lactobacillus rhamnosus strain, piceol, and shikimic acid.

In another aspect, the invention relates to an antimicrobial composition comprising or consisting of piceol and picein wherein piceol is in an excess of picein and optionally further plant derived substance.

In a preferred aspect, the antimicrobial composition comprises a piceol content of at least 200 ppm, more preferably of at least 500 ppm, even more preferably of at least 1000 ppm, and most preferably of at least 3000 ppm. In a further preferred embodiment, the invention provides an antimicrobial composition with a ratio of piceol to picein of at least 2:1, more preferred of at least 4:1, even more preferred of at least 10 : 1, further preferred 40:1 and most preferred of at least 100 : 1. In a preferred embodiment, the percentage of piceol with respect to the sum of piceol and picein amounts to at least 95 wt% and even more preferably at least 97 wt%. Most preferably, 100 wt% piceol is produced.

In yet another preferred aspect, the composition contains at least 75 wt%, preferably at least 80 wt%, further preferably at least 85 wt%, particularly preferably at least 90 wt%, even further preferably at least 95 wt% non-petrochemically-derived carbon based on the total carbon content of the piceol in the composition. Most preferably, all carbon of the piceol in the composition is non-petrochemically-derived carbon, based on the total carbon content of the piceol in the composition.

In a further preferred aspect, the antimicrobial composition contains further plant-derived substances, preferably selected from the group consisting of organic acids such as shikimic acid, quinic acid, citric acid, malic acid, succinic acid, threonic acid and lactic acid.

In a preferred aspect, the antimicrobial composition comprises a Lactobacillus rhamnosus strain, piceol, and shikimic acid.

In yet another aspect, the invention relates to a cosmetic, household, or pharmaceutical product comprising the antimicrobial composition according to the invention.

In a preferred aspect, the product is selected from the group consisting of an oil-in-water or a water-in-oil emulsion, ointment, crème, lotion, and gel. More preferably, the cosmetic or pharmaceutical product is selected from the group comprising or consisting of soap, facial cleanser, exfoliant, mouthwash, hair wash, body wash, hand wash, essence, serum, toner, moisturizer, face mask, multipurpose cleaner, skin cream, lotion, deodorant, solvent, disinfectant, antioxidant, anti-inflammatory, flavoring, fragrance, fragrance masking agent, colorant, antimicrobial antifungal, micellar or fertilizer.

In another aspect, the invention relates to the use of a microorganism, preferably a lactobacillus species for producing an antimicrobial composition, preferably comprising piceol, from picein.

Surprisingly, it was found that using said microorganism in the fermentation of a mixture comprising picein forms a natural antimicrobial composition with a significantly enhanced yield of piceol. In turn, the concentration of picein is significantly decreased compared to its concentration in the natural, untreated plant extract. The invention thus constitutes an environmentally friendly alternative to producing antimicrobial agents by conventional, crude oil-based means. In a preferred embodiment, the use of the microorganism provides a piceol content of at least 200 ppm, more preferably of at least 500 ppm, even more preferably of at least 1000 ppm, and most preferably of at least 3000 ppm piceol. In a further preferred embodiment, the invention provides an antimicrobial composition with a ratio of piceol to picein of at least 2:1, more preferred of at least 4:1, even more preferred of at least 10 : 1, further preferred 40:1 and most preferred of at least 100 : 1. In a preferred embodiment, the percentage of piceol with respect to the sum of piceol and picein amounts to at least 95 wt% and even more preferably to at least 97 wt%. Most preferably, 100 wt% piceol is produced.

In a preferred embodiment, the picein is a picein extract, extracted from a plant selected from the group consisting of Spruce, Salix, Phagnalon Rupestre, Rhodiola Rosea, Poacynum hendersonii, Baccharis magellanica or Vauquelinia.

In a preferred aspect, the picein extract is extracted from a Spruce needle or a Baccharis magellanica plant.

Preferably, said microorganism is a Lactobacillus species, preferably selected from the group consisting of Lactobacillus acetotolerans, Lactobacillus acidophilus, Lactobacillus agrestimuris, Lactobacillus alvei, Lactobacillus amylolyticus, Lactobacillus amylovorus, Lactobacillus animate, Lactobacillus apis, Lactobacillus bombicola, Lactobacillus brevis, Lactobacillus brevisimilis, Lactobacillus casei, Lactobacillus catenefornis, Lactobacillus colini, Lactobacillus corticis, Lactobacillus crispatus, Lactobacillus delbrueckii, Lactobacillus equicursoris, Lactobacillus faeni, Lactobacillus fermentum, Lactobacillus fornicalis, Lactobacillus gallinarum, Lactobacillus gasseri, Lactobacillus gigeriorum, Lactobacillus guizhouensis, Lactobacillus halophilus, Lactobacillus hamsteri, Lactobacillus helsingborgensis, Lactobacillus helveticus, Lactobacillus hominis, Lactobacillus huangpiensis, Lactobacillus iatae, Lactobacillus iners, Lactobacillus insectis, Lactobacillus intermedius, Lactobacillus intestinalis, Lactobacillus isalae, Lactobacillus japonicus, Lactobacillus jensenii, Lactobacillus johnsonii, Lactobacillus juensis, Lactobacillus kalixensis, Lactobacillus kefiranofaciens, Lactobacillus kimbladii, Lactobacillus kitasatonis, Lactobacillus kullabergensis, Lactobacillus laiwuensis, Lactobacillus larvae, Lactobacillus leichmannii, Lactobacillus letivazi, Lactobacillus melliventris, Lactobacillus mobilis, Lactobacillus mulieris, Lactobacillus nasalidis, Lactobacillus panisapium, Lactobacillus paracasei, Lactobacillus paragasseri, Lactobacillus paraplantarum, Lactobacillus pasteurii, Lactobacillus plantarum, Lactobacillus porci, Lactobacillus psittaci, Lactobacillus rennanquilfy, Lactobacillus reuteri, Lactobacillus rizhaonensis, Lactobacillus rhamnosus, Lactobacillus rodentium, Lactobacillus rogosae, Lactobacillus sakei, Lactobacillus salivarius, Lactobacillus selangorensis, Lactobacillus sucicola, Lactobacillus taiwanensis, Lactobacillus terrae, Lactobacillus ultunensis, Lactobacillus vermiforme, Lactobacillus xujianguonis and Lactobacillus xylocopicola.

In a further preferred aspect, the microorganism is selected from the group consisting of Lactobacillus plantarum, Lactobacillus rhamnosus, Lactobacillus brevis, Lactobacillus paraplantarum, Lactobacillus reuteri, Lactobacillus pentosus, Lactobacillus buchneri, Lactobacillus sucicola, Lactobacillus bombi, Lactobacillus gasseri and Lactobacillus rodentium. In yet another preferred aspect, the microorganism is a Lactobacillus rhamnosus strain. In a most preferred embodiment, the natural extract is a spruce needle extract or an extract of the plant Baccharis magellanica and the microorganism according to the invention is a Lactobacillus rhamnosus strain.

In a further preferred embodiment, the use of said microorganism comprises the step of downstream processing of the formed antimicrobial composition, preferably wherein the downstream processing comprises a method selected from the group consisting of centrifugation, crystallization, solid-phase adsorption, filtration, reverse osmosis, precipitation, decantation, liquid/liquid extraction, vacuum distillation, fallfilm distillation, melt crystallization, drying and/or combinations thereof.

In a preferred embodiment, the microorganisms are filtered out of the formed antimicrobial composition. In a further preferred embodiment, heat-inactivation, e.g. by tyndallization or pasteurization, or cell lysis is used as a means for inactivating said microorganisms. Further preferably, the antimicrobial composition is recovered from said mixture.

In a further preferred aspect, the formed antimicrobial composition is substantially non-petrochemical. Said composition is particularly advantageous due to its environmental friendliness and its naturalness, thereby meeting increasing consumer needs and complying with ISO 16128. The composition is considered "substantially non-petrochemical", if at least 75 %, preferably at least 80 %, further preferably at least 85 %, particularly preferably at least 90 %, and even further preferably at least 95 % of its carbon, based on the total carbon content of the piceol in the composition is non-petrochemically-derived carbon. Most preferably, all carbon of piceol in the composition is non-petrochemically-derived carbon based on the total carbon content of the piceol in the composition. Preferably, for the assessment radiocarbon dating is used.

### Brief description of the figures:

### Figure 1

The figure shows the results of the fermentation of a natural plant extract by a microorganism. Briefly, it can be observed that the addition of a *Lactobacillus* strain to both a 1 % and a 2 % all-natural spruce needle extract provided a significant increase in piceol concentration. In turn, picein was found to be significantly decreased compared to the concentration in the natural plant extract.

In the following, the invention and further advantages resulting from it, are explained by means of the examples.

### Example 1: Extraction of fresh spruce needles

Spruce needles and stems from small branches of Picea abies were collected and extracted using water-based extraction. The mixture was stirred, followed by cooling to room temperature. The extracted plant material was separated from the extraction solution by filtration and the extracting solvent was removed by lyophilization. Subsequently, the obtained dry extract was weighed to determine the extraction yields. Picein, piceol and shikimic acid content was quantified by HPLC.

The extraction time and temperature have an impact on the amount and ratio of extracted picein and piceol. Water extraction between 30 and 100 °C is suitable. Accordingly, even an addition of the spruce needles to the fermentation broth could be realized.

| Extract # | Weight of plant material | Weight of water | Extraction temperature | Extraction time | Dry extract yield | Content picein | Content piceol (4-HAP) | Content shikimic acid |
|---|---|---|---|---|---|---|---|---|
| 1 | 57g | 750g | 80°C | 2h | 10.1% | 4.1% | 0.9% | 12.6% |
| 2 | 11g | 200g | 90-100 °C | 3 h | 12.0% | 4,2% | 0.3% | 10.7% |

### Example 2: Extraction of dried spruce needles

Dried spruce needles were extracted using water-based extraction for 2 hours. The mixture was stirred, followed by cooling to room temperature. The extracted plant material was separated from the extraction solution by filtration and the extracting solvent removed by lyophilization. Subsequently, the obtained dry extract was weighed to determine the extraction yields. Picein, piceol, quinic acid and shikimic acid content in the dry extract was quantified by HPLC.

| Weight of plant material | Weight of water | Extraction temperature | Extraction time | Dry extract yield | Content picein | Content piceol (4-HAP) | Content quinic acid | Content shikimic acid |
|---|---|---|---|---|---|---|---|---|
| 51g | 500g | 90-100°C | 2 h | 21.1% | 3.1% | 1.9% | 4.0% | 9.6% |

### Example 3: Extraction of dried spruce needles at different temperatures

20 g of dried spruce needles (Picea abies) were extracted using 150 g of water for 8 hours while stirring at 4 different temperatures. The mixture was treated as described above and the dry extract obtained was weighed to determine the extraction yields. Picein, piceol, quinic acid and shikimic acid content in the dry extract was quantified by HPLC.

| Extract | Extraction temperature | Dry extract yield | Content picein | Content piceol (4-HAP) | Content shikimic acid |
|---|---|---|---|---|---|
| 3.1 | 40°C | 18% | 0.2% | 2.9% | n.d. |
| 3.2 | 60°C | 15% | 0.8% | 2.4% | n.d. |
| 3.3 | 80°C | 15% | 1.6% | 2.0% | 9.2% |
| 3.4 | 100°C | 23% | 1.3% | 1.8% | n.d. |

| | | | | | |
|---|---|---|---|---|---|
| n.d. = not determined | | | | | |

### Example 4: Extraction of Baccharis magellanica

Spruce needles and stems from small branches of the plant Baccharis magellanica were collected and extracted using water-based extraction. The mixture was stirred, followed by cooling to room temperature. The extracted plant material was separated from the extraction solution by filtration and the extracting solvent removed by lyophilization. The dry extract obtained was weighed to determine the extraction yield. Picein, piceol and shikimic acid content was quantified by HPLC.

| Extractant | Weight of plant material | Weight of water | Extraction temperature | Extraction time | Dry extract yield | Content picein | Content piceol (4-HAP) | Content shikimic acid |
|---|---|---|---|---|---|---|---|---|
| Water | 9.5g | 150g | 90-100°C | 3 h | 22.1% | 2.8% | 1.0% | 0.1% |
| Ethanol | 9.8g | 150g | 70-80 °C | 3 h | 25.5% | 6.3% | 0.2% | <0.1% |

### Example 5: In-vitro production of piceol by fermentation using a Lactobacillus strain

| **Sample** | **Picein** | **Piceol** |
|---|---|---|
| 5000 ppm Picein | 4220 ppm | - |
| 5000 ppm Picein after 37° for 3 h | 4640 ppm | - |
| Lactobacillus rhamnosus | - | - |
| Lactobacillus rhamnosus with 5000 ppm Picein after 37° for 3 h | 882 ppm | 1716 ppm |

To investigate the picein conversion to piceol by a Lactobacilli strain, picein was fermented exemplarily with a *Lactobacillus rhamnosus* strain. The bacteria were grown in Man-Rogosa-Sharpe (MRS) medium until exponential growth phase, washed and adjusted to an optical density (OD₆₂₀) of 5 in a simplified medium without glucose. The digestion was performed in a total volume of 1.5 ml in closed tubes without any agitation for 3 h at 37°C.

Surprisingly, as measured by HPLC analysis, an amount of 1716 ppm piceol was produced from 5000 ppm picein. Picein content, in turn, was found to be decreased from 4220 ppm to 882 ppm.

### Example 6: In-vitro production of Piceol from different plant extracts using a Lactobacillus bacterium

| **Sample** | **Picein [ppm]** | **Piceol [ppm]** |
|---|---|---|
| 1 % Extract 1 Control | 354 | 90 |
| 1 % Extract 1 + Lactobacillus | - | 268 |
| 2 % Extract 1 Control | 666 | 176 |
| 2 % Extract 1 + Lactobacillus | 12 | 504 |
| | | |
| 1 % Extract 2 Control | 324 | 28 |
| 1 % Extract 2 + Lactobacillus | - | 200 |
| 2 % Extract 2 Control | 636 | - |
| 2 % Extract 2 + Lactobacillus | 14 | 394 |

*Lactobacillus rhamnosus* was grown in MRS medium until exponential growth phase, washed and adjusted to an optical density (OD₆₂₀) of 5 in a simplified medium without glucose for the actual digestion of the plant extract. Two spruce needle extracts *(Picea abies,* extract 1 and extract 2, see experiment 1 for details), were added at 2% and 1% and incubated at 37°C for 3h. Afterwards, picein and piceol content was determined using HPLC.

Control fermentations containing the plant extract but without addition of any Lactobacillus bacteria were performed in parallel. Measuring the control 1% spruce needle extract 1, the piceol content was found to amount to 90 ppm. In the control 2% spruce needle extract 1, a piceol content of 176 ppm was detected. Surprisingly, after fermentation using a Lactobacillus rhamnosus strain, the piceol content was found to be increased by 198 % to 268 ppm (using the 1% spruce needle extract 1). In the 2% spruce needle extract 1, piceol content was increased by 186 % to 504 ppm. Using extract 2, surprisingly, the piceol content after fermentation was even found to be increased by 614 %, i.e. to 200 ppm.

Picein content was found to be strongly decreased after the fermentation. In the 1% extract, no picein was detected after said fermentation using the Lactobacillus. Moreover, 12 ppm picein were measured in the 2% extract 1. The 2% extract 2 showed 14 ppm picein.

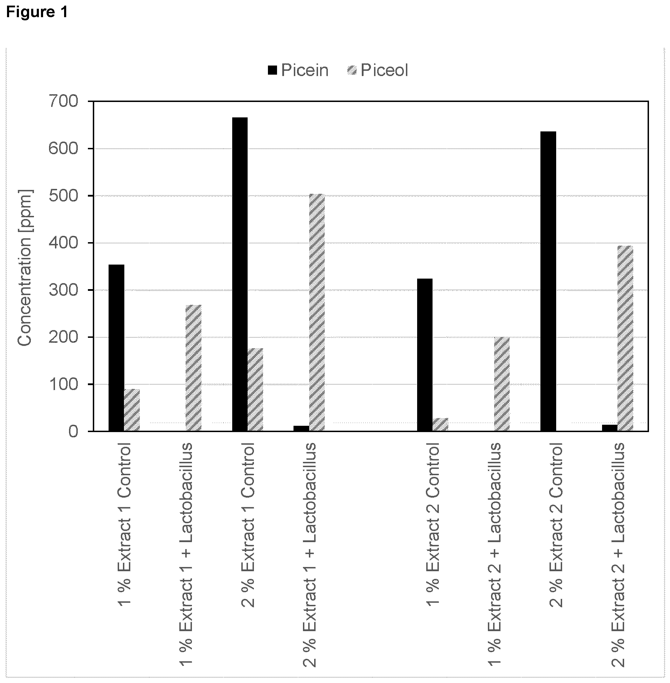

The data is visualized in Figure 1.

### Example 7: Antimicrobial effect of Picein and Piceol

The following preservation efficacy tests (PET) were performed to compare the antimicrobial efficacy of selected compounds in a basic cosmetic formulation (emulsion), prepared as described below.

| **Ingredients** | **INCI** | **w/w %** |
|---|---|---|
| **Phase A** | | |
| Carbopol ETD 2050 ex Lubrizol | Carbomer | 0.2 |
| PCL Solid | Stearyl Heptanoate, Stearyl Caprylate | 4.0 |
| Dragoxat^{®} 89 | Ethylhexyl Isononanoate | 5.0 |
| Lanette 18 ex BASF | Stearyl Alcohol | 2.0 |

| **Phase B** | | |
|---|---|---|
| Water (ultrapure) | Water (Aqua) | ad 100 |
| Dracorin^{®} CE | Glyceryl Stearate Citrate | 3.0 |

| **Phase C** | | |
|---|---|---|
| NaOH 10% aqueous sol. | Sodium Hydroxyide | 0.3 |

| **Phase D** | | |
|---|---|---|
| Test compound | | 1% |
| pH-value | | 5.8 |

The production method comprises heating phases A and B (without Carbopol ETD 2050) separately to approx. 80 °C. Subsequently, Carbopol ETD 2050 is added to phase A, followed by homogenization. Next, phase B is added to phase A and emulsified (Ultra Turrax Stirrer, 2 min, 4000 rpm/min). The mixture is allowed to cool using a reduced stirring speed. The pH value is adjusted with Phase C. Finally, phase D is added and stirred until a homogeneous emulsion is achieved.

The test compounds were incorporated at 1%.

In the PETs the emulsions were inoculated with a defined amount of Candida albicans and Aspergillus niger. The antimicrobial load in the formulation was determined after defined time intervals.

| ***Substance*** | ***Germ** type* | ***Day 0*** | ***Day 2*** | ***Day 7*** | ***Day* 14** | ***Day 28*** |
|---|---|---|---|---|---|---|
| *1% Picein* | C. *albican* s | *210.0* 00 | *140.0* 00 | *140.0* 00 | *89.00* 0 | *36.000* |
| *1% Picein* | *A. niger* | *220.0* 00 | *320.0* 00 | *450.0* 00 | *340.0* 00 | *15.000* |
| *1% Piceol* | C. *albican* s | *210.0* 00 | *<100* | *< 100* | *< 100* | 0 |
| *1% Piceol* | *A. niger* | *300.0* 00 | *56.00* 0 | *1.900* | *1.900* | *<100* |

The table above shows the antimicrobial effect of piceol against the yeast Candida albicans and the fungi Aspergillus niger over the course of 28 days. Picein decreased the concentration of said fungi to a much lesser degree. Similar differences between Picein and Piceol can also be found for bacteria (data not shown).

Accordingly, it can be expected that the fermented plant extract has an improved preservative efficacy compared to the untreated control extract.

## Claims

1. Method for producing an antimicrobial composition, preferably comprising piceol, comprising the following steps:
- providing a growth medium comprising picein and at least one microorganism
- fermenting the mixture comprising picein and said microorganism thereby forming the antimicrobial composition, preferably comprising piceol
- optionally removing the cells of the microorganism from the mixture
- and/or optionally inactivating said microorganism.

2. Method according to claim 1, in which the formed antimicrobial composition comprises a piceol content of at least 200 ppm, preferably of at least 500 ppm, more preferably of at least 1000 ppm, and most preferably of at least 3000 ppm.

3. Method according to any of the claims above, in which the picein is a picein extract, extracted from a plant selected from the group consisting of Spruce, Salix, Phagnalon Rupestre, Rhodiola Rosea, Poacynum hendersonii, Baccharis magellanica or Vauquelinia.

4. Method according to any of the claims above, in which the picein extract is a Spruce needle extract and/or a Baccharis magnellanica extract.

5. Method according to any of the claims above, in which the microorganism is a Lactobacillus species, preferably selected from the group consisting of Lactobacillus plantarum, Lactobacillus rhamnosus, Lactobacillus brevis, Lactobacillus paraplantarum, Lactobacillus reuteri, Lactobacillus pentosus, Lactobacillus buchneri, Lactobacillus sucicola, Lactobacillus bombi, Lactobacillus gasseri and Lactobacillus rodentium.

6. Method according to any of the claims above, in which the picein extract is a Spruce needle extract and/or an extract of the plant Baccharis magellanica and the microorganism is a Lactobacillus rhamnosus strain.

7. Method according to any of the claims above, wherein the formed antimicrobial composition contains at least 75 %, preferably at least 80 %, further preferably at least 85 %, particularly preferably at least 90 %, even further preferably at least 95 % non-petrochemically-derived carbon based piceol, and most preferably wherein all carbon of the piceol in the composition is non-petrochemically-derived carbon based on the total carbon content of the piceol in the composition.

8. Antimicrobial composition, preferably comprising Piceol, obtained or obtainable by a method comprising or consisting of the steps as defined in any of the claims 1 to 7.

9. Antimicrobial composition comprising or consisting of
- at least one microorganism, preferably a Lactobacillus species
- piceol, preferably in an excess amount compared to picein
- optionally picein
- optionally further plant-derived substances.

10. An antimicrobial composition comprising or consisting of
- picein
- piceol, wherein piceol is in an excess of picein
- optionally further plant-derived substances.

11. Composition according to any of the claims 8 to 10, in which the antimicrobial composition comprises a piceol content of at least 200 ppm, preferably at least 500 ppm, even more preferably 1000 ppm, and most preferred 3000 ppm.

12. Composition according to any of the claims 8-11, wherein the composition comprises at least 75 %, preferably at least 80 %, further preferably at least 85 %, particularly preferably at least 90 %, even further preferably at least 95 % non-petrochemically-derived carbon based piceol, and most preferably wherein all carbon of the piceol in the composition is non-petrochemically-derived carbon, based on the total carbon content of the piceol in the composition.

13. Composition according to any of the claims 8-12, in which the further plant-derived substances are preferably selected from the group consisting of organic acids such as shikimic acid, quinic acid, citric acid, malic acid, succinic acid, threonic acid and lactic acid.

14. Cosmetic, household, or pharmaceutical product comprising the antimicrobial composition according to any of previous claims.

15. Use of a microorganism, preferably of a lactobacillus species, for producing an antimicrobial composition from picein.
